Europäisches Patentamt

European Patent Office

Office européen des brevets

(19)

(11) Publication number: **0 233 973**
**B1**

# (12) EUROPEAN PATENT SPECIFICATION

(45) Date of publication of the patent specification:
27.12.90

(51) Int. Cl.⁵: **G01N 33/68**

(21) Application number: 86102505.4

(22) Date of filing: 26.02.86

(54) Mixture of amino acid derivatives, process of producing the mixture and use of the mixture for quantitative determination of the amino acids.

(43) Date of publication of application:
02.09.87 Bulletin 87/36

(45) Publication of the grant of the patent:
27.12.90 Bulletin 90/52

(84) Designated Contracting States:
CH DE FR GB LI NL SE

(73) Proprietor: Hewlett-Packard GmbH, Herrenberger Strasse 130 Postfach 14 30, D-7030 Böblingen(DE)

(72) Inventor: Apffel, James Alex, Dr., 405 Opihikao, Honolulu, HI 96825(US)
Inventor: Schuster, Rainer, Dr., Dekan Fellhauerstr. 22, D-7501 Marxzell-Burbach(DE)

(74) Representative: Patentanwälte Ruff und Beier, Neckarstrasse 50, D-7000 Stuttgart 1(DE)

(56) References cited:
EP-A- 0 138 092
FR-A- 2 088 987
US-A- 3 933 430

INTERNATIONAL BIOTECHNOLOGY LABORATORY, no. 4, December 1983, pages 62-69, Amsterdam, NL; C. TIMOTHY et al.: "HPLC in protein sequencing"
ANALYTICAL CHEMISTRY, vol. 43, no. 7, June 1971, pages 880-882, Columbus, Ohio, US; M. ROTH: "Fluorescence reaction for amino acids"
ANALYTICAL CHEMISTRY, vol. 51, no. 11, September 1979, pages 1667-1674, American Chemical Society, Columbus, Ohio, US; P. LINDROTH et al.: "High performance liquid chromatographic determination of subpicomole amounts of amino acids by precolumn fluorescence derivatization with Omicron-phthaldialdehyde" 1984) 000
JOURNAL OF CHROMATOGRAPHY, vol. 282, 1983, pages 609-618, Elsevier Science Publishers B.V.,

(56) References cited: (continuation)
Amsterdam, NL; S. EINARSSON et al.: "Determination of amino acids with 9-fluorenylmethyl chloroformate and reversed-phase high-performance liquid chromatography"ence derivatization with Omicron-phthaldialdehyde" 1984) 000
JOURNAL OF CHROMATOGRAPHY, vol. 297, 1984, pages 49-61, Elsevier Science Publishers B.V., Amsterdam, NL; H. GODEL et al.: "Measurement of free amino acids in human biological fluids by high-performance liquid chromatography"
INTERNATIONAL BIOTECHNOLOGY LABORATORY, no. 4, December 1983, pages 52-59, Amsterdam, NL; J.M. DI BUSSOLO et al.: "HPLC: A powerful tool for protein analysis"

## Description

The invention described here concerns the chemical derivatization of amino acids. More specifically, a method is disclosed for the preparation, especially of fluorescent derivatives of amino acids suitable for analysis and detection using Liquid Chromatography with reversed phase separation columns. In general the method can be applied to the analysis of amino acids in a variety of matrices such as physiological fluids, pharmaceuticals, foods and beverages.

Today, in many technical areas, the analysis of amino acids is an important requirement. Particularly in the rapidly growing area of biotechnology, this analysis is a critical procedure in the characterization of proteins and peptides. In addition, amino acid analysis is required in the medical field, where characterization of amino acids in biological materials can be useful in diagnostic procedures; in the pharmaceutical field for development and quality control of products; and in the food and beverage area, again for product characterization.

For successful amino acid analysis typically between 18 and 35 amino acids (depending on the matrix) must be separately quantitated in amounts typically between 100 femtomoles ($10^{-13}$ moles) and 1 nanomole ($10^{-9}$ moles) each, preferably with less than 5% sample to sample relative standard deviation, often in the presence of other matrix components, and preferably in less than 1 hour per sample. Modern developments favour high sensitivity and fast analyses.

Most analytical procedures for amino acids are based on liquid chromatography, either in its modern high performance form (HPLC), or as classical medium pressure liquid chromatography. In either case, the major problem in the analysis is the selective and sensitive detection of these compounds. With few exceptions, the amino acids do not show strong optical absorption above 220nm. This precludes the use of ultraviolet/visible spectrophotometric detection at the required sensitivity. Similarly, use of refractive index detection lacks the required sensitivity. For these reasons, chemical derivatization procedures are generally used. The chemical derivatizations serve to tag the compounds with a chemical group so that the resulting product has strong response for either UV/VIS or Fluoresence detection. The derivatization procedure can be performed prior to the chromatographic separation ("precolumn") or after the separation ("postcolumn").

In most cases, the amino group of the amino acid is used as the active site for the chemical derivatization. A major problem arises in the analysis because of the diverse chemical nature and reactivity of the amino acids as a group. Around a common backbone, the amino acids contain such differing functional groups as aliphatic, aromatic, primary amines, secondary amines, carboxyllic acids, amides and thiols. This makes it difficult to find a reagent which will react with all amino acids, yielding comparable sensitivity.

Current chromatographic methods for amino acid analysis can be classified on the basis of the derivatization mode (precolumn or postcolumn) or the detection mode (UV/VIS or Fluorescence) (Amino Acid Analysis, J.M. Rattenbury ed. (Wiley Interscience, New York, 1981))

In terms of the derivatization mode, precolumn derivatization is becoming increasingly more popular because it allows the use of high efficiency, small particle, reversed phase chromatographic columns. Postcolumn derivatization can be used only after separation of the amino acids using ion exchange chromatography, which tends to exhibit poorer chromatographic efficiency and longer analysis time.

The most popular UV/VIS derivatization reactions for amino acids employ either ninhydrin (S. Moore, D.H. Spackmann and W.H. Stein, Anal. Chem. 30 (1958) 1185-1205) or PITC (phenylisothiocyanate) (R.L. Hendrickson and S.C. Meredith, Anal. Bioch., 136 (1984) 65-74). A major disadvantage of UV/VIS derivatization is the relatively low detection sensitivity compared to fluorescence detection. Ninhydrin can be used only postcolumn, but allows the detection of all amino acids. PITC is run precolumn, allows detection of all amino acids, but requires a derivatization procedure which is not amenable to automation.

The most popular fluorescence derivatization reactions utilize either OPA (ortho-phthalaldehyde) (P. Lindroth and K. Mopper, Anal. Chem. 51 (1979) 1667 and M. Roth, Anal. Chem. 43/1971) 880), FMOC (Fluorenylmethylchloroformate) (S. Einarsson, B. Josefsson and S. Lagerkvist, J. Chromatogr. 292 (1983) 609-618) or Dansyl Chloride (Y. Tapuhi, D.E. Schmidt, W. Lindner and B.L. Karger, Anal. Bioch. 15 (1981) 123). OPA is based on a simple, fast, easily automated procedure with high sensitivity, but reacts only with primary amines. Current methods usually react OPA in the presence of mercaptoethanol which results in reagent and products with limited stability.

Both FMOC-Cl and Dansyl Chloride are used in a precolumn mode, deliver high sensitivity and react with primary and secondary amines, but require long reaction times and ancillary procedures such as extractions to remove excess reagent.

Furthermore, all these procedures have difficulty in dealing with the accurate detection of cystine and cysteine. Cystine is a molecule composed of two cysteine molecules joined by a disulfide bridge. Generally a derivative of either cystine or cysteine is detected. However, depending on the sample history, substantial amounts of one may have been converted into the other.

The present invention provides a mixture for use in the determination of amino acids containing fluorescent derivatives of both primary and secondary amino acids as well as remainders of derivating agents from the derivatization process which may be separated by reversed-phase liquid chromatography in such a way that any primary amino acid derivatives elute from the separating column in a sequence with no interspersed elution of any secondary amino acid derivatives and that substantially no remainder from the derivatization process elutes from the chromatographic column at such time as to impede the quantitative determination of any amino acid derivative by wavelength-selective chromatographic detection. This is achieved with the mixture as claimed in claim 1.

In accordance with the present invention there is also provided a process for producing the mixture wherein the sequence of processing steps is adapted to provide for a specific difference between the primary amino acid derivatives and the secondary amino acid derivatives. In a first step the primary amino acids are converted to OPA/MPA derivatives using OPA/MPA dissolved in acetonitrile, and in a succeeding step the secondary amino acids are converted to FMOC derivatives using FMOC dissolved in acetonitrile, all in the presence of suitable buffers for pH adjustment, as claimed in claim 4. The resulting mixture has the required properties.

In accordance with the present invention there is also provided a reagent for use in carrying out the process which has improved long-term stability in storage, as claimed in claim 8.

The present invention furthermore encompasses the use of the claimed mixture for quantitative analysis of amino acids as claimed in claim 9.

From an overall point of view the invention provides a pathway to amino acid analysis having, in particular,
– high sensitivity (500 femtomole/amino acid)
– reliable detection of both primary and secondary amino acids
– total automation capability
– short analysis time (1 hour total analysis cycle).

Preferred embodiments of the invention provide for specific advantages as follows:

In a mixture according to claim 2 cysteine, which does not form a fluorescent product with OPA/MPA if unmodified, may be modified to form a fluorescent OPA/MPA derivative by blocking its thiol group by a reaction with iodoacetic acid and iodoacetamide.

In accordance with claim 5 any cystine contained in an initial mixture may be converted into cysteine by reductive cleaving using mercaptopropionic acid or dithioerythritol as a reducing agent having a mercapto functional group and a higher oxidation potential than cysteine, and the cysteine thiol group may subsequently be blocked using iodoacetic acid or iodoacetamide as a strong alkylating agent so that in the following OPA/MPA derivatization of primary amino acids the cysteine forms a fluorescent OPA/MPA derivative. Any ambiguity in the detection of cystine/cysteine is thus avoided because all cystine is converted to cysteine prior to the derivatization.

It has further been found that, in accordance with claim 6, there is no necessity to perform an extraction of substances from the mixture during the processing because all reagents used are compatible with subsequent chromatographic analysis. Thus the entire process may be carried out in the sample drawing unit of a chromatograph, as claimed in claim 7. It has been found that an apparatus such as is disclosed in co-pending European patent application – publication no.: 0 183 950, publication date: June 11, 1986 – (according to Art. 54 (3) EPC of no relevance regarding the question of inventive step), is particularly suitable for this purpose. In accordance with claim 8 a solution of OPA and MPA in acetonitrile is particularly well suited for use in the claimed process because it has better stability in storage than OPA/MPA formulations previously reported in this field.

The mixture according to the invention may be used for quantitative analysis of amino acids, as claimed in claim 9. In accordance with claim 10 complete separation between the derivatives of the primary amino acids and the secondary amino acids may be had by reversed-phase liquid chromatography. In such an application wavelength-specific analysis is particulary suitable as claimed in claim 11. The column packing material of claim 12 has been found to be advantageous.

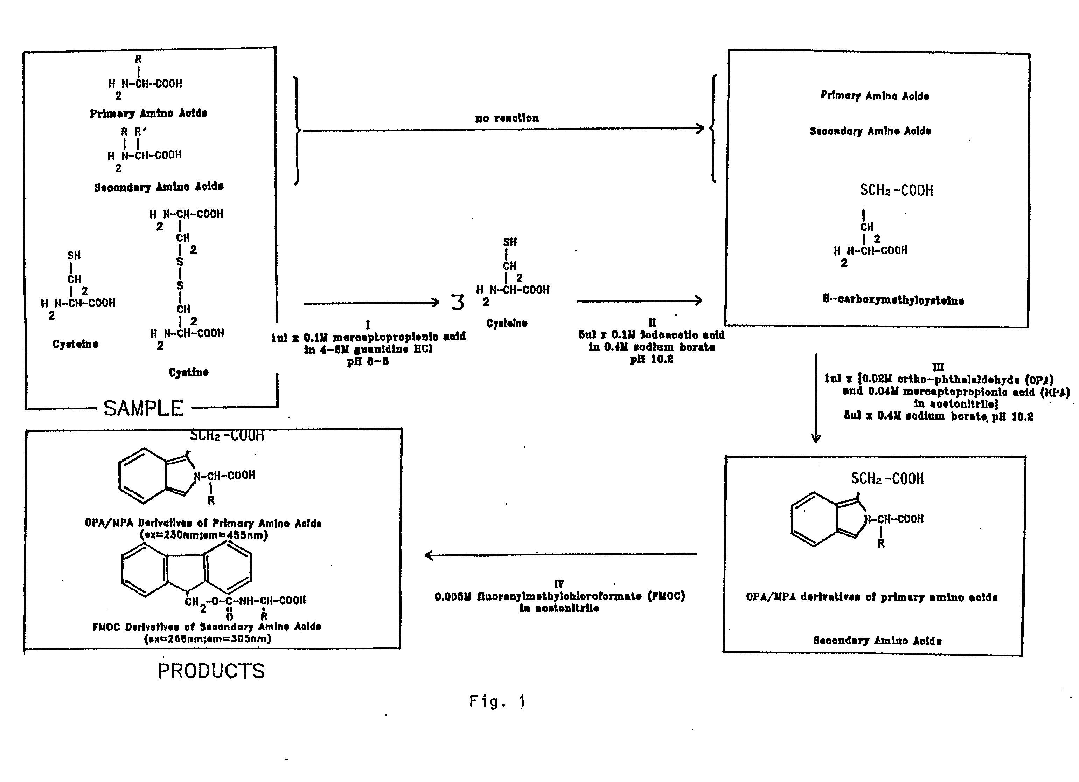

An overview of the entire reaction course according to a preferred embodiment is shown in figure 1. Whereas the invention is substantially based on the subsequent and different derivatization of first the primary amino acids and then the secondary amino acids a preferred embodiment of the process according to the invention can be carried out in a sequence of four reaction steps. Although each reaction step is carried out on the entire analytical sample, not all analytes take part in each reaction. The reaction proceeds as follows:

(I.) In a first facultative reaction step a sample or substrate containing amino acids is mixed with a reductive cleaving agent, preferably mercaptopropionic acid (or dithioerythritol) in urea or guanidine hydrochloride. The effect of this reaction is to transform any cystine into cysteine by reductively cleaving the disulfide bridge. The purpose of this reaction is to convert the cystine into a form (after step II) in which a strongly fluorescent product can be formed e.g. with ortho-phthalaldehyde. By converting cystine to cysteine one reaction can be used to determine the sum quantity of both compounds. Other amino acids are unaffected in this reaction.

(II.) An amount of a blocking agent, preferably iodoacetic acid is then added to the reaction mixture. Iodoacetic acid reacts with the thiols present (i.e. native cysteine and the cysteine newly converted from cystine in step I) to form carboxymethylcysteine. The purpose of this reaction is to convert the cysteine into a form which can form a strongly fluorescent product e.g. with ortho-phthalaldehyde. It is reported (M. Roth, Anal. Chem., 43 (1971) 880) that cysteine does not form a fluorescent product with OPA, but by blocking the thiol group, a fluorescent product can be formed. Other amino acids are unaffected by this reaction.

(III.) The sample (test mixture) is mixed with a suitable buffer such as a volume of borate buffer at pH 10.2 to adjust the pH of the reaction mixture and then a volume of reagent containing ortho-phthalaldehyde (OPA) and mercaptopropionic acid (MPA) (H. Godel, T. Graser, P. Foldi, P. Pfaender and P. Furst, J. Chromatogr. 297 (1984) 49-61), preferably in acetonitrile. In this reaction all primary, but no secondary, amino acids react to form a fluorescent isoindole product which can be separated by reversed phase liquid chromatography and detected using fluorescence (lambda ex = 230 nm, lambda em = 455 nm). Carboxymethylcysteine also forms an isoindole product. All remaining secondary amino acids (principally proline and hydroxyproline) are unaffected by this reaction since OPA only reacts with primary amines. The use of the derivatization reagent dissolved in acetonitrile is a significant improvement since the reagent itself has improved stability in this solution. Previous work has used OPA and MPA (or, more commonly mercaptoethanol) dissolved in borate buffer which is stable maximally for 1-2 weeks.

(IV.) Finally, the reaction mixture (now containing derivatized primary amino acids, free secondary amino acids and excess reagents) is mixed with fluorenylmethylchloroformate (FMOC) preferably dissolved in acetonitrile to form fluorescent products with the secondary amino acids. Like the fluorescent reagent, the products exhibit fluorescence with lambda excitation = 266 nm and lambda emission = 305 nm. Although FMOC reacts with primary amines as well, all primary amino groups have been blocked in the formation of ortho-phthalaldehyde derivatives in step III. The excess reagent FMOC is partially converted to a hydrolysis product resulting in two fluorescent compounds (reagent and hydrolysis product) which can be separated chromatographically from the analytes. The reason all amino acids are not simply reacted with FMOC to form one product is that then the reagent peaks would not be separated easily from the mixture of FMOC primary amino acid derivatives and the reaction mixture would require an extraction step.

After the entire reaction procedure has been completed, the product resulting from the original sample (which contained primary amino acids, including cystine and cysteine, and secondary amino acids) is a mixture of OPA derivatives representing the primary amino acids and FMOC derivatives representing the secondary amino acids. The sum of cysteine and cystine are represented by a OPA product of carboxymethylcysteine. This mixture can be separated using gradient elution reversed phase chromatography preferably using conditions listed below. The use of two separate chemical derivatives additionally allows an added dimension of selectivity, namely that of spectral selectivity.

Although the entire procedure can be used with fluorescence detection for maximal sensitivity, the procedure is also amenable to UV/VIS detection using two simultaneous or sequential detection wavelengths (338 nm for the OPA derivatives, and 266 nm for the FMOC derivatives). Although UV/VIS detection allows poorer detection limits, the detection limits suffice, especially under the preferred conditions and samples mentioned below.

The following is a list of features and advantages of the mixture and the method according to the invention and of preferred embodiments. Wherever possible, it has been pointed out how the advantages compare to other systems.

1. All important amino acids are determined. Both primary and secondary amino acids are determined in a single analysis. This is in comparison to the derivatization of amino acids using ortho-phthalaldehyde alone in which only primary amino acids are determined.

2. Cystine and cysteine are determined. Preferably the sum amount of cystine and cysteine is determined as a single peak. This is to be compared favorably with methods which determine only cystine or cysteine, in which the interconversion of the two is not taken into account. It has to be pointed out that a method which accurately determined both cystine and cysteine individually would be still more attractive.

3. High Sensitivity. The use of fluorescence derivatization and detection allows detection limits between 50 and 100 femtomoles. At this level, the analytical method no longer is the limiting factor in analysis, but rather the background found in samples. This detection limit is to be compared with UV/VIS methods which yield detection limits in the low picomole range.

4. High Selectivity via Spectral information. By using two types of derivatives (OPA and FMOC), a class separation between primary and secondary amino acids can be made based on spectral differences. This can be seen as an extra dimension of selectivity in addition to the chromatographic separation. It simplifies clear differentiation between the two classes of compounds and makes rapid evaluation of chromatographic data easier than methods in which all amino acids are detected under identical conditions.

5. Fast derivatization procedure. As described below, the entire derivatization procedure requires 5-10 minutes. If the procedure is performed manually, this sample preparation time can often become the analytical bottleneck, so a fast procedure is critical. If the procedure is performed on-line, in an automated fashion (see point 9), a fast derivatization procedure allows high sample throughput.

6. Fast analysis time. In addition to the fast derivatization procedure, the analysis is fast due to the use of small particle reversed phase chromatographic columns and gradient elution techniques. Using an automated derivatization procedure, the entire sample cycle is 35 minutes. This is to be compared with methods using post-column derivatization in which ion-exchange chromatography is used, requiring 1-1.5 hours per sample. Additionally, reversed phase chromatographic columns tend to be more stable than ion-exchange columns.

7. Room temperature reaction. In addition to being fast, the derivatization reaction takes place at ambient temperatrue thus requiring no additional hardware for thermostatic control. This is to be critically compared with the phenylisothiocyanate (PITC) method in which a long reaction requires elevated temperatures and a special hardware module for operation.

8. Homogeneous, compatible reagents. All the reagents used in the procedure are mutually compatible. In other words, there is no cross reaction or interference between one reaction and another. This is an essential feature of the method. Although the individual reaction steps have been described in one form or another, modifications have had to be made to ensure this compatibility. For example, OPA is typically dissolved in a borate buffer containing 1-5 % methanol, but it was found that methanol may interfere with the FMOC reaction, so that the OPA solvent was preferably changed to 100 % acetonitrile which does not interact with FMOC. The homogeneous character of the reaction (i.e., the entire reaction can be carried out in a single vessel, adding one reagent at a time, but never removing reagents) makes the system ideal for automation. Again this should be compared with the PITC method in which excess reagent must be removed under vacuum, or the FMOC method in which the excess reagent is extracted.

9. Easily Automated. Due to the points described above, the derivatization procedure can be automated using a computer controlled HPLC autosampler system such as that described in the copending European patent application no. 85 113 154.0, reference is made to this application. It should be noted that although this is a great advantage in terms of sample throughput and automation capability, the method is still valid and useful in a manual mode and does not depend on the automation aspect for success.

10. Use of Mercaptopropionic Acid in OPA reaction. The use of this reagent has been described before, but is still not completely accepted. The use of mercaptopropionic acid in place of mercaptoethanol in the OPA reaction causes higher fluorescence yield and results in stable fluorescence products, whereas mercaptoethanol yields unstable products with half-lives measured in minutes.

11. Stable reagent solutions. The preferred use of acetonitrile as a solvent for the ortho-phthalaldehyde/mercaptopropionic acid reagent and for the FMOC reagent results in reagent solutions which have improved stability. This is a very important improvement, because using a buffer as solvent (in the absence of some stabilizer) results in a reagent which is only stable for a matter of weeks. This is a critical point if the reagents are to be prepared in large batches and later sold and distributed.

12. Use of mercaptopropionic acid for cleavage of cystine disulfide bond. As described in points 8 and 9, the total reaction scheme consists of a series of mutually compatible reactions. A preferred and important point in question is the use of mercaptopropionic acid for the reductive cleavage of the cystine disulfide bond. It is important that this is the same chemical that is later used in the OPA reaction, because the thiol forms a functional group of the product. If mercaptopropionic acid is used in place of another thiol acid (such as mercaptoethanol, dithioerythritol or dithiothritol), it is avoided that multiple products would be formed for each amino acid. Furthermore, after the cleavage of the disulfide bonds, the thiol groups of cysteine are blocked such as by use of iodoacetic acid preventing them from playing the roll of the thiol in the OPA reaction (as opposed to the role of the amino acid).

13. Non-toxic, -mutaganic or -carcinogenic reagents. As opposed to reagents such as nitroben-zooxadiazole chloride (NBD-Cl) or nitrooxadiazole fluoride (NBD-F), the reagents are relative safe in a hygienic sense.

Following are specific and preferred details of the reaction steps involved in the above procedure. Further, there were given acceptable ranges for reagent volumes and concentrations These ranges are shown parenthetically next to the optimal or typical values. The procedures described assume 1 microliter starting sample volume, although this volume can be varied if corresponding variations are made in the reagent volumes. The reagents are not exotic and can be obtained from various vendors so long as the purity is sufficient. After the addition of each reagent, the mixtures are thoroughly mixed.

I. One microliter of sample is mixed with 5 microliters (1-10 microliters) of 0.1 M (0.01-0.5 M) analytical grade mercaptopropionic acid dissolved in 4 M (3-6M) reagent grade urea or guanadine hydrochloride at pH 6-8. Alternatively, 0.4 M (0.1-0.4 M) reagent grade sodium borate buffer adjusted to pH 10.2 (9.5-10.5) with sodium hydroxide can be used as solvent. In place of mercaptopropionic acid, 0.1 M (0.01-0.5 M) dithioerythritol can be used. The reaction proceeds at room temperature (20- 30 deg. C) within 1 to 5 minutes.

II. To the mixture from step I is added 0.1 M (0.05-0.3 M) iodoacetic acid dissolved in 0.4 M (0.1-0.4 M) sodium borate buffer adjusted to pH 10.2 (9.5-10.5) with sodium hydroxide. The exact concentration of iodoacetic acid should be approximately as a 10 fold molar excess to the amount of cysteine expected in the mixture (including that converted from cystine). The reaction proceeds at room temperature in 1 to 5 minutes. In place of iodoacetic acid, iodacetamide can be used, resulting in a different product than carboxymethylcysteine and with different chromatographic properties.

III. To the reaction mixture resulting from step II (or to one microliter of initial sample), add 5 microliters (1-10 microliters) of 0.4 M (o.1-0.4 M) sodium borate buffer adjusted to pH 10.2 (9.5-10.5) and 1 microliter (1-5 microliter) of a solution containing 0.02 M (0.01-0.1 M) ortho-phthalaldehyde and 0.04 M (0.01-0.1 M) mercaptopropionic acid dissolved in acetonitrile. The reaction proceeds at room temperature in 1 to 5 minutes.

IV. To the reaction mixture resulting in step III, add 1 microliter (1-5 microliter) of a solution containing 0.01 M (0.005-0.05 M) fluorenylmethylchloroformate dissolved in acetonitrile The reaction proceeds at room temperature in 1-5 minutes.

The entire reaction mixture is then injected directly into a High Performance Liquid chromatograph with the following conditions:

Analytical Conditions

Column: 200 x 2.1 mm i.d. 5 μm Hypersil ODS (octa decylsilane modified)
Column Temperature: 35 deg. C.

Mobile Phases:
A: 2.4 g/L sodium acetate
0.25 % tetrahydrofuran
in bidistilled water
B: 80 % acetonitrile
20 % 0.1 M sodium acetate in bidistilled water.
Initial flow rate: 0.22 ml/min
Gradient program:
AT T=0; A=98 % B=2 %
AT T=10; A=82 % B=18 %
AT T=18; A=71 % B=29 %
AT T=20; A=55 % B=45 %
AT T=22; A=48 % B=52 %
AT T=24; A=00 % B=100 %
AT T=29; A=00 % B=100 %
AT T=31; A=98 % B=2 % (times in minutes)

Detection parameters

Fluorescence Detection:

OPA derivatives (T=0-21 minutes)

Excitation wavelength = 230 nm; Slit width=5nm
Emission wavelength=455 nm; Slit width=5nm

FMOC Derivatives (T=21-25 minutes)

Excitation wavelength = 266 nm; slit width=5nm
Emission wavelength = 305 nm; slit width=5nm
UV/VIS Detection:

OPA derivatives:

Signal: 338nm, Bandwidth: 10nm
Reference: 390nm Bandwidth: 20 nm
FMOC Derivatives:

Signal: 266nm Bandwidth: 4nm
Reference: 349nm Bandwidth: 6nm
These HPLC analytical conditions are given as typical without being limiting.

## Claims

1. A mixture for use in the determination of amino acids containing fluorescent derivatives of both primary and secondary amino acids as well as remainders of chemicals used in the derivatization process, characterized in that any primary amino acid derivative present is an OPA/MPA derivative, that any secondary amino acid derivative present is an FMOC derivative and that the mixture contains acetonitrile.

2. A mixture as in claim 1, characterized in that any cysteine derivative present is an OPA/MPA derivative of a cysteine derivative having its thiol group blocked by blocking groups resulting from a reaction with iodoacetic acid and iodoacetamide.

3. A mixture as in claim 1 or claim 2 in the form of an aqueous solution.

4. A process for producing the mixture as in claim 1 from a test mixture containing primary and secondary amino acids, characterized by the steps of first mixing the test mixture with a buffer and a solution of ortho-phthalaldehyde (OPA) and mercaptopropionic acid (MPA) in acetonitrile in any sequence to obtain a first mixture and then mixing the first mixture with a buffer and a solution of fluorenylmethylchloroformate (FMOC) in acetonitrile in any sequence.

5. A process as in claim 4, characterized by the steps of preparing the test mixture from an initial mixture containing cystine by first mixing the initial mixture with a buffer and mercaptopropionic acid or dithioerythritol as a reducing agent having a mercapto functional group and a higher oxidation potential than cysteine in any sequence to convert cystine into cysteine by reductively cleaving cystine disulfide bonds, thereby obtaining an intermediate mixture, and then mixing the intermediate mixture with a buffer and iodoacetic acid or iodoacetamide as a strong alkylating agent in any sequence to block free cysteine thiol groups, thereby obtaining the test mixture.

6. A process as in claim 4 or claim 5, characterized in that no part of any reagent used in a processing step and no part of any reaction product resulting from a processing step is selectively removed from the mixture before a succeeding processing step is performed.

7. A process as in any one of claims 4 through 6, characterized in that the mixture is contained in a sample drawing unit of a chromatograph during the entire processing.

8. A solution of OPA and MPA in acetonitrile for use in the process as in any one of claims 4 through 7.

9. A use of the mixture as in any one of claims 1 through 3 for quantitative analysis of amino acids.

10. A use as in claim 9, characterized in that at least a sample of the mixture is passed through a reversed-phase liquid chromatography column to cause elution of any derivatives of primary amino acids from the column in a sequence with no interspersed elution of any secondary amino acid derivatives.

11. A use as in claim 10, characterized in that during a first period of time the elute from the column is irradiated with light of at least a first wavelength specific to OPA/MPA derivatives of primary amino acids, that during a second period of time the elute from the column is irradiated with light of at least a second wavelength specific to FMOC derivatives of secondary amino acids, and that a detection of light is performed for quantitative analysis of amino acids.

12. A use as in claim 10 or 11, characterized in that the column is packed with octadecylsilane-modified silicagel.

## Patentansprüche

1. Mischung zur Verwendung bei der Bestimmung von Aminosäuren enthaltend fluoreszierende Derivate von sowohl primären als auch sekundären Aminosäuren sowie auch Reste von Chemikalien, die im Derivatisierungsverfahren verwendet wurden, dadurch gekennzeichnet, daß jedes vorhandene primäre Aminosäurederivat ein OPA/MPA-Derivat ist, daß jedes vorhandene sekundäre Aminosäurederivat ein FMOC-Derivat ist, und daß die Mischung Acetonitril enthält.

2. Mischung nach Anspruch 1, dadurch gekennzeichnet, daß jedes vorhandene Cysteinderivat ein OPA/MPA-Derivat eines Cysteinderivats ist, dessen Thiolgruppen durch Schutzgruppen blockiert sind, die von einer Reaktion mit Jodessigsäure und Jodacetamid resultieren.

3. Mischung nach Anspruch 1 oder Anspruch 2 in Form einer wässrigen Lösung.

4. Verfahren zur Herstellung der Mischung nach Anspruch 1 aus einer Testmischung, die primäre und sekundäre Aminosäuren enthält, gekennzeichnet durch die Schritte, daß zuerst die Testmischung mit einem Puffer und einer Lösung von ortho-Phthalaldehyd (OPA) und Mercaptopropionsäure (MPA) in Acetonitril in beliebiger Reihenfolge gemischt wird, um eine erste Mischung zu erhalten, und dann die erste Mischung mit einem Puffer und einer Lösung von Fluorenylmethylchlorformiat (FMOC) in Acetonitril in beliebiger Reihenfolge gemischt wird.

5. Verfahren nach Anspruch 4, gekennzeichnet durch die Schritte, daß die Testmischung aus einer Ausgangsmischung, die Cystin enthält, hergestellt wird, indem zuerst die Ausgangsmischung mit einem Puffer und Mercaptopropionsäure oder Dithioerythrit als Reduktionsmittel, das eine funktionelle Mercaptogruppe besitzt und ein höheres Oxidationspotential als Cystein, in beliebiger Reihenfolge gemischt wird, um durch reduktives Spalten der Cystin-Disulfidbrücken Cystin in Cystein zu überführen, wobei eine intermediäre Mischung erhalten wird, und dann die intermediäre Mischung mit einem Puffer und Jodessigsäure oder Jodacetamid als starkes Alkylierungsmittel in beliebiger Reihenfolge gemischt wird, um freie Cystein-Thiolgruppen zu blockieren, wobei die Testmischung erhalten wird.

6. Verfahren nach Anspruch 4 oder Anspruch 5, dadurch gekennzeichnet, daß kein Teil eines Reagens, das in einem Herstellungsschritt verwendet wird und kein Teil eines Reaktionsprodukts, das aus einem Herstellungsschritt resultiert, selektiv aus der Mischung entfernt wird, bevor ein nachfolgender Verfahrensschritt durchgeführt wird.

7. Verfahren nach einem der Ansprüche 4 bis 6, dadurch gekennzeichnet, daß die Mischung während des ganzen Verfahrens in einem Probenahmeelement eines Chromatographen enthalten ist.

8. Lösung von OPA und MPA in Acetonitril zur Verwendung in dem Verfahren nach einem der Ansprüche 4 bis 7.

9. Verwendung der Mischung nach einem der Ansprüche 1 bis 3 zur quantitativen Analyse von Aminosäuren.

10. Verwendung nach Anspruch 9, dadurch gekennzeichnet, daß mindestens eine Probe der Mischung durch eine Umkehrphasen-Flüssigchromatographiesäule geschickt wird, um die aufeinanderfolgende Elution der Derivate von primären Aminosäuren aus der Säule zu bewirken, ohne daß dazwischen sekundäre Aminosäurederivate eluiert werden.

11. Verwendung nach Anspruch 10, dadurch gekennzeichnet, daß während eines ersten Zeitraums das Eluat aus der Säule mit Licht von mindestens einer ersten Wellenlänge, die für OPA/MPA-Derivate von primären Aminosäuren spezifisch ist, bestrahlt wird, daß während eines zweiten Zeitraums das Eluat aus der Säule mit Licht von mindestens einer zweiten Wellenlänge, die für FMOC-Derivate von sekundären Aminosäuren spezifisch ist, bestrahlt wird, und daß eine Lichtbestimmung zur quantitativen Analyse von Aminosäuren durchgeführt wird.

12. Verwendung nach Anspruch 10 oder 11, dadurch gekennzeichnet, daß die Säule mit Octadecylsilan-modifiziertem Silicagel gepackt ist.

**Revendications**

1. Mélange utilisé pour la détermination d'amino-acides contenant des dérivés fluorescents des amino-acides aussi bien primaires que secondaires, ainsi que les restants des produits chimiques utilisés dans le procédé de formation du dérivé caractérisé en ce que tout dérivé d'amino-acide primaire présent est un dérivé OPA/MPA, en ce que tout dérivé d'amino-acide secondaire présent est un dérivé FMOC et en ce que le mélange contient de l'acétonitrile.

2. Mélange selon la revendication 1, caractérisé en ce que tout dérivé de cystéine présent est un dérivé OPA/MPA d'un dérivé de cystéine dont le groupe thiol est bloqué par des groupes de blocage provenant d'une réaction avec l'acide iodacétique et un iodacétamide.

3. Mélange selon la revendication 1 ou 2, sous forme d'une solution aqueuse.

4. Procédé de production du mélange selon la revendication 1, à partir d'un mélange de test contenant des amino-acides primaires et secondaires, caractérisé en ce qu'on mélange d'abord le mélange de test avec un tampon et une solution d'ortho-phtalaldehyde (OPA) et un acide mercapto-propionique (MPA) dans l'acétonitrile dans un ordre quelconque pour obtenir un premier mélange et, ensuite, on mélange ce premier mélange avec un tampon et une solution de chloroformiate de fluorénylméthyle (FMOC) dans l'acétonitrile dans un ordre quelconque.

5. Procédé selon la revendication 4, caractérisé en ce qu'on prépare le mélange de test à partir d'un mélange initial contenant de la cystine, en mélangeant d'abord le mélange initial avec un tampon et l'acide mercaptopropionique ou le dithioérythritol à titre d'agent réducteur comportant un groupe mercapto fonctionnel et ayant un potentiel d'oxydation plus élevé que celui de la cystéine dans un ordre quelconque pour convertir la cystine en cystéine par un clivage réducteur des liaisons de disulfure de cystine, pour obtenir ainsi un mélange intermédiaire et, ensuite, on mélange le mélange intermédiaire avec un tampon et l'acide iodacétique ou l'iodacétamide à titre d'agent alkylant fort dans un ordre quelconque pour bloquer les groupes thiol de la cystéine libre pour obtenir ainsi le mélange de test.

6. Procédé selon la revendication 4 ou 5, caractérisé en ce qu'on ne soutire pas sélectivement de partie quelconque d'un réactif utilisé dans un stade opératoire ni de partie d'un produit de réaction résultant quelconque du stade opératoire avant d'effectuer le stade de traitement suivant.

7. Procédé selon l'une quelconque des revendications 4 à 6, caractérisé en ce que le mélange est contenu dans une unité de prélèvement d'échantillon d'un chromatographe pendant la totalité du traitement.

8. Solution de OPA et MPA dans l'acétonitrile pour utilisation dans le procédé selon l'une quelconque des revendications 4 à 7.

9. Utilisation du mélange selon l'une quelconque des revendications 1 à 3, pour l'analyse quantitative d'amino-acides.

10. Utilisation selon la revendication 9, caractérisée en ce qu'on fait passer au moins un échantillon du mélange à travers une colonne de chromatographie liquide en phase inversée pour provoquer l'élution des dérivés quelconques des amino-acides primaires de la colonne en séquence sans élution intercalée de dérivés d'amino-acides secondaires quelconques.

11. Utilisation selon la revendication 10, caractérisée en ce que, pendant un premier intervalle de temps, l'éluat de la colonne est irradié avec la lumière d'au moins une première longueur d'onde spécifique aux dérivés OPA/MPA d'amino-acides primaires, en ce que, pendant un second intervalle de temps, l'éluat de la colonne est irradié avec la lumière d'une seconde longueur d'onde spécifique aux dérivés FMOC d'amino-acides secondaires et en ce qu'on effectue une détection de lumière en vue d'une analyse quantitative des amino-acides.

12. Utilisation selon la revendication 10 ou 11, caractérisée en ce que la colonne est garnie de silicagel modifié par l'octadécylsilane.

EP 0 233 973 B1

**SAMPLE**

R
|
H N–CH–COOH
2

**Primary Amino Acids**

R R′
| |
H N–CH–COOH
2

**Secondary Amino Acids**

SH
|
CH
| 2
H N–CH–COOH
2

**Cysteine**

H N–CH–COOH
2 |
CH
| 2
S
|
S
|
CH
| 2
H N–CH–COOH
2

**Cystine**

no reaction →

**I**
1ul x 0.1M mercaptopropionic acid
in 4–6M guanidine HCl
pH 6–8

→ 3

SH
|
CH
| 2
H N–CH–COOH
2

**Cysteine**

**II**
5ul x 0.1M iodoacetic acid
in 0.4M sodium borate
pH 10.2

→

**Primary Amino Acids**

**Secondary Amino Acids**

SCH₂ –COOH
|
CH
| 2
H N–CH–COOH
2

**S–carboxymethylcysteine**

**III**
1ul x [0.02M ortho–phthalaldehyde (OPA)
and 0.04M mercaptopropionic acid (MPA)
in acetonitrile]
5ul x 0.4M sodium borate, pH 10.2

↓

SCH₂ –COOH

N–CH–COOH
|
R

**OPA/MPA derivatives of primary amino acids**

**Secondary Amino Acids**

**IV**
0.005M fluorenylmethylchloroformate (FMOC)
in acetonitrile

←

**PRODUCTS**

SCH₂ –COOH

N–CH–COOH
|
R

**OPA/MPA Derivatives of Primary Amino Acids**
(ex=230nm;em=455nm)

CH₂–O–C–NH–CH–COOH
‖ |
O R

**FMOC Derivatives of Secondary Amino Acids**
(ex=266nm;em=305nm)

Fig. 1